# EUROPEAN PATENT SPECIFICATION

(11) **EP 1 501 931 B1**
(45) Date of publication and mention of the grant of the patent: **18.11.2009**
(21) Application number: 03749984.5
(22) Date of filing: 06.05.2003
(51) Int. Cl.: C12N 15/11, A61K 48/00

(54) **CHIMERIC SNRNA MOLECULES CARRYING ANTISENSE SEQUENCES AGAINST THE SPLICE JUNCTIONS OF THE DYSTROPHIN GENE AND THEIR THERAPEUTIC APPLICATIONS**
CHIMÄRE SNRNA MOLEKÜLE MIT ANTISENSE-SEQUENZEN GEGEN DIE "SPLICE JUNCTIONS" DES DYSTROPHIN-GENS UND IHRE THERAPEUTISCHEN VERWENDUNGEN
MOLECULES DE SNARN CHIMERES PORTANT DES SEQUENCES ANTISENS CONTRE LES JONCTIONS D'EPISSAGE DU GENE DE LA DYSTROPHINE ET APPLICATIONS THERAPEUTIQUES ASSOCIEES

(30) Priority: 08.05.2002 IT RM20020253
(43) Date of publication of application: 02.02.2005
(73) Proprietor: UNIVERSITA' DEGLI STUDI DI ROMA "LA SAPIENZA", 00185 Roma (IT)
(72) Inventor: BOZZONI, I. Dipt. Genetica e Biologia Molecolare, Piazzale Aldo Moro, 5 I-00185 Roma (IT); DE ANGELIS, F.G. Dipt. Genetica e Biologia Molecolare, Piazzale Aldo Moro, 5 I-00185 Roma (IT)
(74) Representative: Capasso, Olga
(86) International application number: PCT/IT2003/000273
(87) International publication number: WO 2003/095647

(56) References cited:
- EP-A- 1 191 097
- GORMAN ET AL.: "Restoration of Correct Splicing of Thalassemic ß-Globin Pre-mRNA by modified U1 snRNAs" J BIOL CHEM, vol. 275, no. 46, 17 November 2000 (2000-11-17), pages 35914-35919, XP002261981 cited in the application
- M. MATSUO: "Duchenne and Becker Muscular Dystrophy: From Gene Diagnosis to Molecular Therapy" IUBMB LIFE, vol. 53, no. 3, March 2002 (2002-03), pages 147-152, XP009021242
- DEUTEKOM VAN J C T ET AL: "Antisense-induced exon skipping restores dystrophin expression in DMD patient derived muscle cells" HUMAN MOLECULAR GENETICS, OXFORD UNIVERSITY PRESS, SURREY, GB, vol. 10, no. 15, 2001, pages 1547-1554, XP002250908 ISSN: 0964-6906
- MANN C J ET AL: "ANTISENSE-INDUCED EXON SKIPPING AND SYNTHESIS OF DYSTROPHIN IN THE MDX MOUSE" PROCEEDINGS OF THE NATIONAL ACADEMY OF SCIENCES OF USA, NATIONAL ACADEMY OF SCIENCE. WASHINGTON, US, vol. 98, no. 1, 2 January 2001 (2001-01-02), pages 42-47, XP001160821 ISSN: 0027-8424 cited in the application
- SUTER D ET AL: "DOUBLE-TARGET ANTISENSE U7 SNRNAS PROMOTE EFFICIENT SKIPPING OF AN ABERRANT EXON IN THREE HUMAN BETA-THALASSEMIC MUTATIONS" HUMAN MOLECULAR GENETICS, OXFORD UNIVERSITY PRESS, SURREY, GB, vol. 8, December 1999 (1999-12), pages 2415-2423, XP000973208 ISSN: 0964-6906 cited in the application
- DE ANGELIS FERNANDA GABRIELLA ET AL: "Chimeric snRNA molecules carrying antisense sequences against the splice junctions of exon 51 of the dystrophin pre-mRNA induce exon skipping and restoration of a dystrophin synthesis in Delta 48-50 DMD cells." PROCEEDINGS OF THE NATIONAL ACADEMY OF SCIENCES OF THE UNITED STATES OF AMERICA. UNITED STATES 9 JUL 2002, vol. 99, no. 14, 9 July 2002 (2002-07-09), pages 9456-9461, XP002261982 ISSN: 0027-8424
- BRUN C ET AL: "U7 snRNAs induce correction of mutated dystrophin pre-mRNA by exon skipping." CMLS CELLULAR AND MOLECULAR LIFE SCIENCES, vol. 60, no. 3, March 2003 (2003-03), pages 557-566, XP009021235 ISSN: 1420-682X

### SEQUENCE LISTING

<110> Università degli Studi di Roma La Sapienza
<120> CHIMERIC snRNA MOLECULES CARRYING ANTISENSE SEQUENCES AGAINST THE SPLICE JUNCTIONS OF THE DYSTROPHIN GENE AND THEIR THERAPEUTIC APPLICATIONS
<130> 30130
<160> 16
<170> PatentIn version 3.1
<210> 1
   <211> 32
   <212> DNA
   <213> artificial sequence
<220>
   <223> anti 24+2A
<400> 1
   gtatgagaaa aaatgagatc ttgggcctct gc 32
<210> 2
   <211> 33
   <212> DNA
   <213> artificial sequence
<220>
   <223> ANTI 24+2B
<400> 2
   cttctgcttg atggcagggg agataccatg atc 33
<210> 3
   <211> 29
   <212> DNA
   <213> artificial sequence
<220>
   <223> U1cas A
<400> 3
   cctggtacca tgagatcttg ggcctctgc 29
<210> 4
   <211> 27
   <212> DNA
   <213> artificial sequence
<220>
   <223> U1cas B
<400> 4
   cctctcgagc tgactttctg gagtttc 27
<210> 5
   <211> 35
   <212> DNA
   <213> artificial sequence
**<220>**
   <223> U2mA
<400> 5
   aaaggctgtc ttcgcatgcg ctcgccttcg cgccc 35
<210> 6
   <211> 36
   <212> DNA
   <213> artificial sequence
<220>
   <223> U2mB
<400> 6
   tggctatctc tcgagtatca gtttaatatc tgatac 36
<210> 7
   <211> 41
   <212> DNA
   <213> artificial sequence
<220>
   <223> U2X
<400> 7
   ctcgagagat agccaaaagg ctgtcttcgc atgcgctcgc c 41
<210> 8
   <211> 50
   <212> DNA
   <213> artificial sequence
<220>
   <223> U2BP
<400> 8
   aaaaagaaga aaaagaaaaa ttagaaacta tcagtttaat atctgatacg 50
<210> 9
   <211> 34
   <212> DNA
   <213> artificial sequence
<220>
   <223> U2cas up
<400> 9
   ctagctagcc aggccttcgg cttcttcgac tggg 34
<210> 10
   <211> 34
   <212> DNA
   <213> artificial sequence
<220>
   <223> U2cas down
<400> 10
   ctagctagcg cgcgtcacag gactcgtgca agcc 34
<210> 11
   <211> 24
   <212> DNA
   <213> artificial sequence
<220>
   <223> U1a
<400> 11
   atcaagcaga aggtatgaga aaaa 24
<210> 12
   <211> 23
   <212> DNA
   <213> artificial sequence
<220>
   <223> U2a
<400> 12
   gtttctaatt tttctttttc ttc 23
<210> 13
   <211> 22
   <212> DNA
   <213> artificial sequence
<220>
   <223> E46F
<400> 13
   gaatttgttt tatggttgga gg 22
<210> 14
   <211> 22
   <212> DNA
   <213> artificial sequence
<220>
   <223> E54F
<400> 14
   cttttatgaa tgcttctcca ag 22
<210> 15
   <211> 20
   <212> DNA
   <213> artificial sequence
<220>
   <223> E47F
<400> 15
   ttactggtgg aagagttgcc 20
<210> 16
   <211> 26
   <212> DNA
   <213> artificial sequence
<220>
   <223> E52R
<400> 16
   ttcgatccgt aatgattgtt ctagcc 26

## Description

The present invention concerns chimeric snRNA molecules carrying antisense sequences against the splice junctions of the dystrophin gene and their therapeutic applications. In particular, the invention concerns chimeric molecules able to mask the splice junctions of mutated exons thus inducing their skipping from the mature mRNA. The resulting transcript will then produce a shorter but functional dystrophin protein, these molecules can be used in the gene therapy of many forms of Duchenne Muscular Dystrophy (DMD).

DMD is an X-linked recessive disorder which affects 1 in every 3500 males. It is characterized by the absence of the cytoskeletal dystrophin (427 KDa protein) that in turn produces a severe and progressive muscle deterioration. Most DMD mutations consist of deletions and point mutations in the 2.5 Mb dystrophin gene which introduce stop codons and mutations in the 2.5 Mb dystrophin gene which introduce stop codons and consequently premature translation termination. In a milder form of myopathy, Becker Muscular Dystrophy (BMD), deletions inside the gene produce in-frame mRNAs and consequently shorter but semi-functional dystrophin proteins (1). Since a third of DMD cases result from de novo mutation (2,3), the disease can never be eradicated through genetic screening and counseling. The need to develop treatment strategies for this disorder is evident. One involves the transplantation of normal myoblasts into the muscle tissues lacking this protein (4,5), while others try to restore the correct expression of dystrophin through a gene therapy approach that delivers full-length or mini cDNA copies of dystrophin into cells having the mutated gene (6-8). Although this approach is very promising, several problems still remain to be solved, such as size capacity and transducing activity of the vector and immune response to the "therapeutic" gene (9).

Another approach of gene therapy is based on the fact that internal in-frame deletions in the protein produce only mild myopathic symptoms; therefore, it should be possible, by preventing the inclusion of specific mutated exon(s) in the mature dystrophin mRNA, to restore a partially corrected phenotype. In this respect, it is interesting to note that restoration of dystrophin levels as low as 30% are sufficient for substantial therapeutic benefit (10).

Exon-specific skipping has been accomplished by the extracellular delivery of synthetic 2'-O-methyl antisense oligonucleotides raised against specific splicing junctions (11-13) or exonic enhancers (10). The interaction of the antisense RNA with the corresponding target sequence should mask the utilization of specific splice sites or prevent the binding of enhancer factors during the splicing reaction and determine the skipping of the neighboring mutated exon(s) so as to produce an in-frame dystrophin mRNA, the ultimate effect being the production of a shorter but functional dystrophin protein. A significant drawback to the synthetic $oligonucleotide approach is that it would require periodic administrations.

To circumvent this problem, the authors of the invention have developed vectors able to express *in vivo,* in a stable and continuous fashion, large amounts of chimeric RNAs containing antisense sequences. This strategy was tested on the human deletion of exons 48-50, but experts could apply it also to other exons of the dystrophin gene. In this case, a premature termination codon is produced in exon 51; if skipping of this exon is obtained, the result would be an in-frame mRNA.

The antisense sequences cloned in snRNA vectors can be designed to pair with any region of a cellular transcript. In principle, if there are sequences that have to be recognized by specific cellular machineries their masking by antisense RNAs should interfere with specific processes.

Since snRNAs are localized in the nucleus, they can affect only nuclear processes.

Antisense RNAs can affect splicing by interacting with two types of sequences:
a) *splice sites.* Antisense RNAs against splice sites can induce, during splicing, the skipping of a specific exon.
b) *splicing enhancers.* Many human exons, of large size or with relatively weak flanking splice sites, contain sequences that promote their utilization, the so called exonic splicing enhancer (ESEs). Such sequences are recognized by serine/arginine (SR)-rich proteins that promote exon utilization in tissues where they are expressed. Antisense RNAs that obscure such splicing enhancers can be designed so that the exon is skipped and not included in the mature mRNA. With respect to the targeting of 3' and 5' splice site sequences that can cause less predictable skipping of additional adjacent exons, the knock out of exonic enhancers produces a very specific and more reproducible effect only on the flanking introns.

Targeting of splice sites or exonic enhancers can be applied in all those cases where the skipping of a specific mutated exon can restore the functionality of the mRNA (such as in the case of dystrophin mutations).

Since more than half of human genes encode transcripts that undergo alternative splicing, general methods to control the expression of particular alternatively spliced isoforms from specific genes would be useful in a wide variety of applications. Antisense RNAs against specific splicing junctions can control the type of alternative spliced mRNA produced and of the corresponding protein.

Antisense RNAs can be also applied to interfere with the following processes:
1) 3'end formation of mRNAs. This process requires a complex protein apparatus that by recognizing specific conserved sequences present in the 3' UTR determines cleavage of the nascent transcript and addition of the polyA tail. Masking the sequences recognized by the cleavage/polyadenylation components or the cleavage site should prevent 3' end formation thus producing read-through to the downstream sequences. Read-through RNAs are rapidly driven to the degradative pathway since they lack the polyA tail. The antisense strategy in this case should drastically decrease the abundance of a specific mRNA. This strategy is also useful in those cases where alternative cleavage/polyadenylation sites are present on the same gene. Skipping of one of the alternative sites can control the type of transcript produced.
2) Editing

RNA editing is a post-transcriptional nuclear process consisting in the site-specific modification of the mRNA. Specific factors, which interact with the RNA, are responsible for such modifications. Also in this case masking of the substrate should prevent editing to occur thus altering the coding capacity of the mRNA.

In order to obtain effective "therapeutic" RNA molecules *in vivo,* several important parameters were considered. Not only must the genes for these RNAs be cloned under efficient promoters producing high levels of expression but, in addition, the RNA context in which the therapeutic RNA is embedded should provide stability and specific subcellular localization. The, last point is a crucial one since in the cell RNAs are sorted to specific cellular locations (nucleus, nucleolus, cytoplasm, free and polysome-bound RNPs, etc.) and efficient activity of the therapeutic molecule can be obtained only if co-localization with the target is ensured (15,33,34). Small cellular RNAs have been advantageously used as vectors since they are normally transcribed from strong promoters (poIII or poIIII) and because they allow the delivery of the chimeric constructs into specific cellular compartments (15,26,30,32,33).

The authors have utilized different small nuclear RNAs and their corresponding genes in order to express in the nucleus chimeric molecules carrying antisense sequences complementary to exon 51 splice junctions and they have tested their activity in myoblasts from DMD patients with a deletion of exons 48-50. Splice junction is a region across the splice site and comprises the sequences upstream and downstream from the site. The results obtained showed that the combination of antisense molecules against the 5' and 3' splice junctions induced efficient skipping of exon 51 and partial rescue of dystrophin synthesis. The choice of the carrier RNAs was dictated by the possibility of co-localizing the antisense molecules with their target RNA. Two of the selected RNAs, U1 and U2, are known to participate in the splicing reaction, thus increasing the probability that the antisense molecule is delivered to the compartment where the dystrophin pre-mRNA is localized. In this technique the nucleoplasmic U7 snRNA was previously utilized to express antisense molecules *in vivo* and to direct exon skipping of a mutant beta-globin gene (32). However, the U7 snRNA gene promoter is known to be less active (17,35,36). The authors of the invention have selected U1 and U2 RNAs instead, because they direct antisense molecules specifically and selectively in the splicing compartment, thus improving co-localization with the target molecule. In addition, the vectors of the invention hit both the 5' and 3' splicing sites. Lastly, the globin gene represents a system of expression and synthesis that differs from that of dystrophin.

Analysis of cell lines derived from a dystrophic patient, immortalized with a SV40 large T, and stably transduced with retroviral vectors carrying the different constructs, indicates that efficient skipping is obtained when two antisense molecules specific for the 5' and 3' splice junctions of exon 51 are co-expressed in the same cell. A lower effect is observed when single antisense molecules are utilized. In those ceiis where exon 51 skipping is observed, rescue of dystrophin synthesis is obtained.

It is therefore an object of the present invention to provide a gene encoding a modified human U1 snRNA wherein a the U1 5' gene fragment transcribing the region of U1 that is single-stranded comprises an antisense sequence complementary to the 5' and 3' splice junctions of at least one exon sequence of the dystrophin pre-mRNA, so that at least one exon sequence of the dystrophin pre-mRNA is skipped during the splicing process that converts the pre-mRNA into the mature mRNA.

Preferably the modified U1 snRNA is further modified such that that the U1 gene fragment transcribing the RNA sequence interacting to the U1 associated 70k protein is deleted.

In the invention, at least exon 51 of the dystrophin pre-mRNA may be skipped.

It is another object of the invention to provide a modified human U1 snRNA transcribed by the gene of the invention as described above.

It is a further object of the invention to provide a vector for stable and efficient expression in target cells expressing dystrophin transcribing the modified human U1 snRNA as described above. The vector may be a retroviral, adenoviral or adeno-associated viral vector. Preferably the vector is suitable for transfer to stem cells or to muscle fiber cells.

The invention also provides a gene, a modified human U1 snRNA or a vector of the invention as described above for use in a method of treatment of the human or animal body by therapy.

Also provided by the invention is a gene, a modified human U1 snRNA or a vector according to the invention as described above for use in the treatment of Duchenne Muscular Dystrophy.

The invention further provides use of a gene, a modified human U1 snRNA or a vector according to the invention as described above in the manufacture of a medicament for use in the treatment of Duchenne Muscular Dystrophy.

The vector segments able to ensure the stable and efficient expression of the gene for the snRNA modified according to the invention method will be selected by a field expert according to the target cell. If muscle fiber cells are the target, they comprise retroviral, adenoviral adeno-associated, lentiviral, etc. portions.

Advantageously, compared with the previous technique that uses antisense oligonucleotides, the skipping of the mutated exon and the restoration of dystrophin synthesis are characteristics that the transduced cells acquire permanently, thus avoiding the need for continuous administration to achieve a therapeutic effect. The vectors of the invention therefore represent a tool for the development of strategies for the in vivo delivery of therapeutic RNAs, by engineering the antisense RNA within the vectors that efficiently transduce in muscle fiber cells, like, for example, the vectors derived from retroviruses, such as lentiviruses, adenoviruses or adeno-associates.

The invention is described below in relation to non-limiting experimental examples and in reference to the following figures:

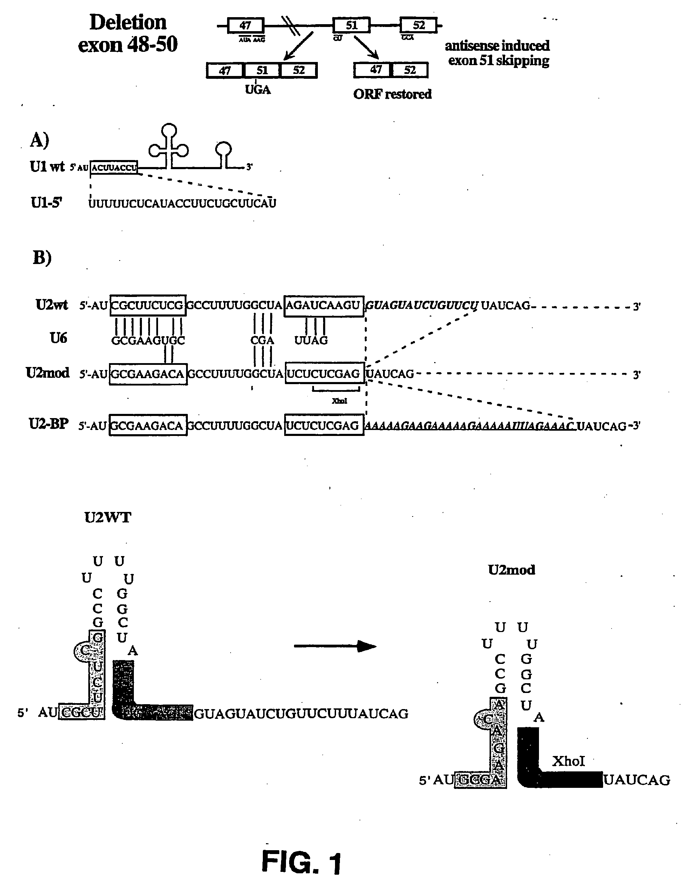

Figure 1 Schematic representation of the dystrophin mutation utilized in this study. Patient 491 A has a deletion encompassing exon 48 through 50. Top: the splicing of the pre-mRNA transcribed from this mutant gene is shown (exons are represented by boxes and introns by lines): an out-of-frame mRNA with a stop codon in exon 51 is produced. Right: the deletion of exon 51 from the mRNA produces a functional mRNA coding for a 217-amino acid shorter protein. Antisense RNAs directed against exon 51 splicing junctions are also schematically represented with respect to their target substrate. Panel A) The U1-5' construct was obtained by replacing the region complementary to 5' splice junctions (boxed nucleotides) with an antisense sequence against the 5' splice junction of exon 51. Panel B) Structure of the U2 derivatives. The U2snRNA was initially converted into the U2mod construct that was, in turn, modified to give U2BP. The sequence of the 5' portion of the wild type and U2 snRNA, derivatives (nucleotides 1-52) is shown in the upper panel together with that of U6 snRNA complementary nucleotides. The boxed regions indicate the nucleotide substitutions introduced, while the pairing to U6 are indicated by bars. The 14 nucleotide long region of U2, indicated in italics, containing the branch-site pairing region, was deleted in U2 mod. In U2BP this region was replaced with a 28-nucleotide long sequence complementary to the 3' region containing the branch site of intron 50 (underlined). The modifications of U2 mod were made so as to maintain the overall secondary structure (see the schematic representation in the lower panel), while preventing the pairing with the U6 snRNA.

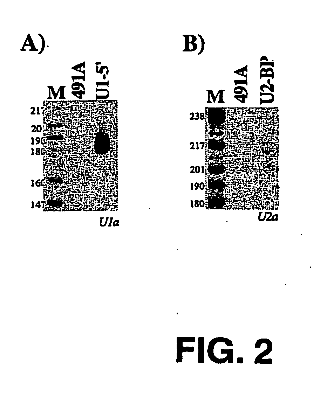

Figure 2. *In vivo* expression of the antisense constructs. 8 micrograms of total RNA, extracted from mixed cell populations transduced with the different chimeric pBabe vectors (indicated above the lanes) and from control untransduced cells (lanes 491A), were run on 6% polyacrylamide/7M urea gels and electroblotted onto nylon membranes. Hybridizations were carried out with ³²P-labelled oligonucleotides specific for each antisense RNA, as indicated below each panel. Lane M: molecular weight marker (pBR322 plasmid DNA, Mspl digested).

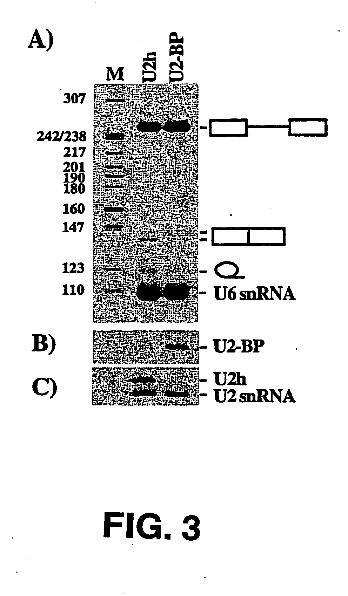

Figure 3. The effect of the U2 antisense construct on splicing. The oocytes were injected with plasmids containing U2h or U2-BP, as indicated in the Materials and Methods section. The nuclei of the individual oocytes were isolated manually and the RNA extracted. The samples were separated on 6% polyacrylamide/7M urea gels and transferred. Direct autoradiography of the filter shows ³²P-labelled RNAs (Panel A), while the hybridizations containing specific probes allow analysis of the expression of the injected plasmids (Panels B and C). In B) a specific antisense probe was used, and in C) a probe for U2 snRNA. U2h snRNA accumulates in the oocytes chiefly as a precursor a few nucleotides longer (band U2h). The different splicing products are shown at the side. Lane M: pBR322 digested with Mspl.

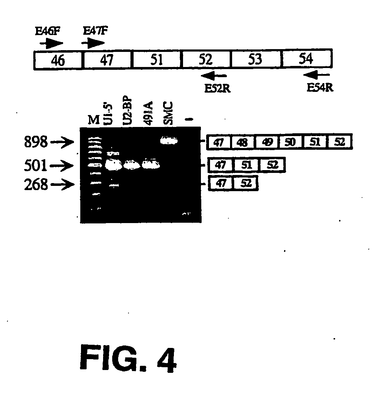

Figure 4. Analysis of the effects of the antisense construct on the splicing pattern of dystrophin mRNA. The upper part of the panels shows the oligos utilized for the nested RT-PCR reactions on the 491A dystrophin pre-mRNA. RT-PCR was performed with the external oligos; after 40 rounds of amplification, nested PCR was performed with the internal ones. The products of amplification from untreated (lanes 491 A) and transduced (the name of the specific antisense construct is indicated above each lane) cell lines were run on 2% agarose gels in parallel with a RNA-minus control (lanes-) and with the products of amplification obtained on RNA from a wild type skeletal muscle cell line (lane SMC). The 501 nucleotide-long amplification product corresponds to the unskipped 491A RNA, while the 268 nucleotide-long fragment corresponds to the skipped mRNA. In lane SMC, the 898 nucleotide amplification product has the size expected for a wild type dystrophin mRNA. Lane M: 100bp DNA ladder (Invitrogen).

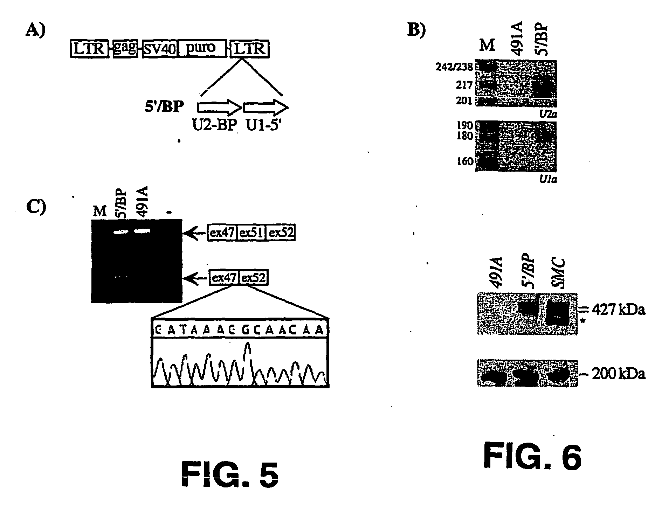

Figure 5. Analysis of the effects of double antisense constructs on the splicing pattern of dystrophin mRNA. Panel A): schematic representation of pBabe puro retroviral construct. The antisense expressing cassettes were cloned head-to-tail in the 3' LTR and forward orientation. Vector 5'/BP contains constructs U2BP and U1-5'. Panel B): Northern blot analysis was performed on 8 micrograms of total RNA extracted from 491A cells untransduced (lanes 491A) or transduced with the 5'/BP vector (Lane: 5'/BP). Hybridization was carried out with ³²P-labelled oligonucleotides indicated below. Lane M: molecular weight marker (pBR322 plasmid DNA, Mspl digested). Panel C): nested RT-PCR was carried out on RNA from control untransduced cells (lane 491A) and from cells transduced with the double construct 5'/BP. A control amplification was carried out in the absence of RNA (lane -). Lane M: 100bp DNA ladder (Invitrogen). A schematic representation of the unskipped and skipped products is shown on the side of the gel. In the lower part of the panel, the sequence of the shorter RT-PCR products shows the precise skipping of exon 51.
Figure 6. Western blot analysis. 100 micrograms of total proteins extracted from 491A cells untransduced (lanes 491A) or transduced with the 5'/BP vector were run on a 6% polyacrylamide-SDS gel in parallel with proteins extracted from wild type skeletal muscle cells (lane SMC). The size of the dystrophin protein is indicated on the side.

### Materials and Methods

### Plasmid constructs

Clone U1-5' was obtained by inverse PCR on plasmid pHU1-ID, containing the entire U1 RNA human gene, with the oligos:
ANTI 24+2A, 5'-GTATGAGAAAAAATGAGATCTTGGGCCTCTGC-3';
ANTI 24+2B 5'-CTTCTGCTTGATGGCAGGGGAGATACCATGATC-3'.

The U1 expression cassette was constructed by inverse PCR, with oligonucleotides:
U1cas A, 5'- CCTGGTACCATGAGATCTTGGGCCTCTGC-3';
U1 cas B, 5'-CCTCTCGAGCTGACTTTCTGGAGTTTC-3'.

In this construct, the U1 snRNA coding region (from nt 3 to161) was replaced with the KpnI, StuI and Xho restriction sites.

The human U2snRNA gene (Fig. 1B), including the promoter and the 3' downstream sequence, was used to generate by inverse PCR the U2mod construct with oligonucleotides:
U2mA, 5'- AAAGGCTGTCTTCGCATGCGCTCGCCTTCGCGCCC-3';
U2mB, 5'- TGGCTATCTCTCGAGTATCAGTTTAATATCTGATAC-3'.

The U2BP construct was derived from U2mod by inverse PCR with oligonucleotides:
U2X: 5'- CTCGAGAGATAGCCAAAAGGCTGTCTTCGCATGCGCTCGCC-3';
U2BP: 5'-AAAAAGAAGAAAAAGAAAAATTAGAAACTATCAGTTTAATATCTGATACG-3'.

### Retroviral constructs

### Single antisense constructs

U2BP was amplified by direct PCR using oligonucleotides:
U2cas up 5'-CTAGCTAGCCAGGCCTTCGGCTTCTTCGACTGGG-3';
U2cas down 5'-CTAGCTAGCGCGCGTCACAGGACTCGTGCAAGCC-3'.

The resulting fragments were cloned in the forward orientation into the Nhel site of the pBabe puro retroviral vector (23). The BamHI fragments containing the entire chimeric construct U1-5' were inserted in the pBabe Nhel site modified with BgIII adaptors.

### Double antisense constructs

The 5'/BP construct was made by cloning the BamHI fragment containing the U1-5'coding region inside the U2BP plasmid. This construct was amplified with oligos U2cas up and U1cas down and cloned in the Nhel site of the pBabe vector.

### Packaging Cell line

The Phoenix packaging cell line (hftp//www.stanford.edu/nolan) was cultured in Dulbecco's modified Eagle's medium supplemented with 10% FCS and 1 % penicillin-streptomycin (GIBCO/BRL). Retroviral particles were collected from Phoenix cells transiently transfected with 4 □g of the various pBabe constructs using LipofectAmine plus (GIBCO/BRL) and were used to transduce the cell line of the patient with DMD.

### Cell Culture

The Duchenne derived biopsy (491A) was put in culture and maintained in RPMI supplemented with 15% FCS, 1% penicillin-streptomycin and 1% glutamine (GIBCO/BRL). Cells were propagated by standard trypsinization and seeded directly in a collagen coated dish (Rat tail collagen Type 1, BD). Infection of the cells was carried out in the absence of any antibiotic. One day before infection cells were plated at a density of approximately 5X10⁵ cells per 100mm dish. Two rounds of infections were performed with the different viral supernatants; 48 h after infection the cells were divided and puromycin was added at the final concentration of 1 mg/ml (Sigma).

### RNA Preparation and analysis

Total cellular RNA was prepared using the Ultraspec RNA isolation system (BIOTECX Laboratories, INC.) according to the manufacturers protocol. For Northern Blot analysis 8□g of total RNA was loaded on 6% polyacrylamide -7M urea gels blotted and hybridized with the following antisense specific probes:
U1a 5'-ATCAAGCAGAAGGTATGAGAAAAA-3';
U2a 5'GTTTCTAATTTTTCTTTTTCTTC-3'.

RT-PCR was carried out with 200ng of total RNA for 40 cycles of amplification using Access RT-PCR system (Promega). The first amplification was performed with primers:
E46F, 5'-GAATTTGTTTTATGGTTGGAGG-3 ;
E54F, 5'-CTTTTATGAATGCTTCTCCAAG-3'.

Two microliters of the RT-PCR product was used as template in a 50µl secondary nested PCR using the oligos:
E47F, 5'-TTACTGGTGGAAGAGTTGCC-3' (exon 47) and
E52R 5'-TTCGATCCGTAATGATTGTTCTAGCC-3' (exon 52).

The nested PCR was carried out for 30 cycles. Nested products were analyzed on 2.5% agarose gel and specific bands purified for sequence analysis using the QIAquick Gel extraction Kit (Qiagen). DNA sequencing was carried out by M-medical using the BigDye Terminator Cycle Sequencing Ready Reaction kit (PE Applied Biosystems) and analyzed on an ABI Prism 377 Sequencer (PE Applied Biosystems).

### Microinjections in X. laevis oocytes

The AdMI pre-RNA is 253 nucleotides lone and comprises a part of the late principle transcript of the Adenovirus. It is obtained by in vitro transcription of the pAdML-Di1 plasmid digested with Sca 1 (38). The RNAs of ³²P-labelled AdML and U6 (1.5x10⁶ and 0.4x10⁶ cpml/µl, respectively) were co-injected in oocytes that had received the previous night the U2-BP or U2h plasmids (4 ng/oocyte). After incubation for 1 h, the RNA was extracted and analyzed on 6% polyacrylamide-urea gel.

### Western Blotting analysis

Cultured cells were washed twice with ice-cold phosphate saline buffer (PBS) and then lysed for 40'at 4°C in a buffer containing 500 mM Tris-HCl pH 8, 150 mM NaCl, 100 mM NaF, 1 mM EGTA, 1.5mM MgCl₂, 1% Triton X-100, 10% glycerol and supplemented with various protease inhibitors (leupeptin, PMSF, apoprotein). After clearing by centrifugation at 15.300 rpm for 10', protein content was quantified by BIO-RAD Protein Assay (BIO-RAD). 100 micrograms of proteins were separated onto a 6% polyacrylamide gel containing 0.1% SDS, 150 mM Tris HCl pH 8.8 and transferred to polyscreen PVDF transfer membrane (Nen Life Science). After treatment with 5% nonfat dry milk in Tris-buffered saline Tween (TBS-T), the membrane was incubated with NCL-DYS1 mouse monoclonal antibody (Novocastra Laboratories Ltd) diluted 1:100 for 60', followed by three washes in TBS-T buffer and incubation with a horseradish peroxidase-linked secondary antibody (1:1000; Amersham Pharmacia Biotech) for 45'. Detection of proteins was carried out with super signal chemiluminescent substrate (PIERCE).

### Results

### Construction of the antisense chimeric RNAs

From the available cases the authors selected one, patient 491 A, having a deletion encompassing exons 48, 49 and 50 (Ricci and Galluzzi, personal communication). In this case, skipping of exon 51 would produce an in frame mRNA coding for a protein 210 amino acids shorter than the wild type dystrophin (Fig.1 top).

The U1 snRNA was engineered so as to substitute 8 nucleotides at its 5' terminus with a 24 nucleotide-long sequence complementary to the corresponding region across the splice junction of exon/intron 51 (construct U1-5'; Fig. 1A). This choice was dictated by the fact that the 5' terminal region of U1 snRNA is normally in a single stranded conformation and is involved in the recognition of the 5' splice junction of pre-mRNAs (14). The expression of the chimeric RNA is directed by the polymerase II-dependent promoter of the U1 snRNA gene which was previously shown to drive high levels of transcripts (15). It is also easily possible to remove the region of the U1 snRNA that binds to protein 70 KDa which is involved in the activation of the 3' splice site of the upstream intron and/or insert in both U1 and U2 the double antisenses of the molecule.

The other type of RNA vector used is the U2 snRNA. This RNA is normally involved in the splicing reaction since it recognizes the intronic branch site sequence and directs the entry, in the spliceosome, of the catalytic U6 snRNA (21,22). Both the recognition of U2 snRNA for the branch site and its interaction with U6 snRNA are mediated by base pair interactions. In the authors' design, a U2 derivative (U2mod) was first produced with mutations in the region pairing to U6 snRNA. Nucleotide substitutions were made in such a way that the secondary structure of the U2 snRNA would not be affected (Fig. 1B). The lack of interaction with the U6 snRNA should ensure that the modified U2 snRNA would not interfere with the cellular splicing apparatus and would not recruit the spliceosome on the intron to which it is paired. U2 mod was subsequently modified so that the branch site pairing region was substituted with 28 nucleotides complementary to the branch site and 3' region of intron 50, giving rise to construct U2-BP (Fig. 1B).

All the described constructs were cloned separately or in couples in the 3' LTR (U3 region) of the pBabe-puro retroviral vector and the viral particles were obtained by transfecting plasmid DNAs into the Phoenix packaging cell line (23).

### Transduction of antisense constructs into DMD-derived cells and analysis of their expression

The cells from a muscle biopsy of the 491A DMD patient were put in culture and immortalized by retroviral-mediated integration of a wild-type SV40 large-T antigen (24). The immortalized cells were infected with recombinant retroviral particles and stable integrations were selected in puromycin-containing medium.

Expression of the antisense RNAs was controlled by Northern analysis on the total cell population grown in the presence of puromycin. Figure 2 shows that all the constructs express the chimeric RNAs at fairly good levels

Experiments of *X*. *laevis* oocyte microinjection showed that all the constructs described above are expressed at high levels and that all of them specifically localized in the nuclear compartment. These results demonstrate that the modifications introduced in the different snRNAs do not affect either their overall stability or their subcellular localization.

It is known that the U1 construct does not interfere with endogenous cell functions (30, 31). To determine whether an excess of the modified form of U2 snRNA interfered with the efficiency of the splicing mechanism, splicing products from a control pre-mRNA were accumulated. ³²P-labelled AdML pre-mRNA (see Materials and Methods section) was injected into the nuclei of *X*. *laevis* oocytes previously injected with the U2h or U2-BP gene. The ³²P-labelled U6 snRNA was co-injected as an internal control. Figure 3A shows that no reduction in the splicing efficiency is observed when the construct with the antisense U2 molecule is overexpressed in the nuclei compared with the overexpression of the wild-type U2h snRNA. Northern blot analysis was also performed on the oocytes to verify the expression of the injected DNAs. Panel B) shows the expression of U2-BP, and Panel C) the expression of control U2h snRNA. The U2h-specific expression is revelled by the presence of a pre-U2 snRNA that accumulates specifically in the oocytes (37). Therefore, the overexpression of U2-BP does not interfere with the efficiency of the splicing mechanism.

### Analysis of the mRNA produced in response to antisense treatment

Mixed cell populations expressing different types of single chimeric constructs were analyzed by RT-PCR for the presence or absence of exon 51 in the mature mRNA. Figure 4 shows the gel analysis of the RT-PCR products obtained with specific couples of oligos as specified at the top of each panel. Of the first series of cells tested, only U1-5' gave a faint band corresponding to the size of a product in which exon 51 was skipped. If compared with the amount of the unskipped product, it appears that less than 10% of the mRNA was deprived of exon 51.

To determine whether the efficiency of correction increases in the presence of antisense RNA against both splice junctions, an additional construct was made in which couples of antisense constructs were cloned in tandem inside the pBabe retroviral vector (Fig.5A). Also in this case immortalized DMD cells were infected with recombinant retroviral particles and stable integrations were selected in puromycin-containing medium.

The expression of these double antisense constructs was tested in vivo by Northern analysis. Fig.5B shows that head-to-tail cloning does not interfere with efficient expression of both chimeric RNAs.

The DMD myoblasts carrying the double antisense constructs were then analyzed for exon 51 skipping by RT-PCR. Figure 5C shows that construct 5'/BP produces the appearance of a band corresponding to the size of a skipped product (217 nucleotides long). Sequence analysis of the gel-purified band indicated that the skipped product contains an exact fusion of exon 47 with exon 52. These data support the previous hypothesis that both splice junctions should be masked by antisense sequences in order to have efficient skipping of exon 51. In the 5'/BP construct, the amount of skipped product is in the order of 30-40%.

### Rescue of dystrophin synthesis

Immortalized 491A cells transduced with the double antisense constructs were analyzed for dystrophin synthesis by Western analysis. One hundred micrograms of total proteins were run on a 6% polyacrylamide gel and, after blotting, were reacted with dystrophin specific antibodies. In comparison to untreated DMD (lane 491A), the cells expressing the antisense constructs show rescue of dystrophin synthesis (Fig. 6). The levels of dystrophin are fairly good if compared with those present in the control of skeletal muscle cells (lane SMC). The faster migration band (indicated by a dot) should represent a specific degradation product of dystrophin. The lack of size difference observed between normal and induced dystrophin on the Western blot was expected, because removal of exons 48 to 51, which codes for 210 amino acids, would not significantly alter the migration of such a large (427-Kda) protein in a polyacrylamide gel. The lower panel in Figure 6 show a control of Western blot analysis on the same gel with anti-myosin antibodies.

References
1) Koenig, M., et al. (1989) Am J Hum Genet. 45, 498-506.
2) Hodgson, S.V. & Bobrow, M. (1989) Br Med Bull. 45, 719-744.
3) Zatz, M., Lange, K. & Spence, M. A. (1977) **1**, 759.
4) Gussoni, E., Blau, H. M. & Kunkel, L. M. (1997) Nat Med. 3, 970-977.
5) Partridge, T.A., et al. (1989) Nature 337,176-9
6) Wang, B., Li, J. & Xiao, X. (2000) Proc Natl Acad Sci U S A 97, 13714-13719.
7) Campeau, P., et al. (2001) Gene Ther. 8, 1387-1394.
8) Cho, W. K., et al. (2000) Hum Gene Ther 11, 701-14
9) Chen, H. H., et al. (1997) Proc Natl Acad Sci USA 94, 1645-50
10) van Deutekom, J. C., et al. (2001) Hum Mol Genet. 10,1547-1554.
11) Mann, C. J., et al. (2001) Proc Natl Acad Sci U S A. 98, 42-47.
12) Dunckley, M. G.; et al. (1998) Hum Mol Genet. 7, 1083-1090.
13) Wilton, S. D., et al. (1999) Neuromuscul Disord. 9, 330-338.
14) Zhuang, Y. & Weiner, A. M. (1986) Cell. 46, 827-835.
15) Michienzi, A., Prislei, S. & Bozzoni, I. (1996) Proc Natl Acad Sci U S A. 93, 7219-7224.
16) German. L., et al. (1998) Proc Natl Acad Sci U S A. 95, 4929-4934.
17) Grimm, C., Stefanovic, B. & Schumperli, D. (1993) EMBO J. 12,1229-1238.
18) Galli, G., et al. (1983) Cell. 34, 823-828.
19) Bond, U. M., Yario, T. A. & Steitz, J.A. (1991) Genes Dev. 5, 1709-1722.
20) Spycher, C., et al. (1994) Nucleic Acids Res. 22, 4023-4030.
21) Datta, B. & Weiner, A.M. (1991) Nature 352, 821-824.
22) Sun, J.S. & Manley, J.L. (1995) Genes Dev. 9, 843-854.
23) Morgestem, J. P. & Land, H. (1990) Nucleic Acids Res. 18, 3587-3596.
24) Berghella, L., et al. (1999) Hum Gene Ther. 10, 1607-1617.
25) Cech, T. R. (1989) Biochem Int. 18, 7-14.
26) Buonomo, S.B., et al. (1999) RNA 5, 993-1002.
27) Crooke, S. T. (1998) Antisense Nucleic Acid Drug Dev. 8, 133-134.
28) Kole, R. & Sazani, P. (2001) Curr Opin Mol Ther 3, 229-234.
29) Sierakowska, H., et al. (1996) Proc Natl Acad Sci U S A 93, 12840-12844.
30) Gorman, L., Mercatante, D. R. & Kole, R. (2000) J Biol Chem 275, 35914-35919
31) Michienzi, A., et al. (2000) Proc Natl Acad Sci U S A. 97, 8955-8960.
32) Suter, D., et at. (1999) Hum Mol Genet. 8, 2415-2423.
33) Bertrand, E., et al. (1997) RNA 3, 75-88.
34) Sullenger, B. A. & Cech, T. R. (1993) Science 262, 1566-1569.
35) Bimstiel, M. L. & Schaufele, F. (1988) in Structure and function of major and minor small nuclear ribonucleoprotein particles, ed. Birnstiel, M. L. (Springer, Berlin), pp. 155-182.
36) Baserga, S.J. & Steitz J. A. (1993) in The RNA world (Gesteland R.F., and Atkins J.F., eds.), pp. 359-381. Cold Spring Harbor Press, Plainview. NY.
37) You, C-H, Ares, M Jr & Weiner, A M (1985) Cell 42, 193-202.
38) Fragapane P., et al. (1992) Mol. Cell. Biol. 12, 1117-1125.

## Claims

1. A gene encoding a modified human U1 snRNA wherein the U1 5' gene fragment transcribing the region of U1 that is single-stranded comprises an antisense sequence complementary to the 5' and 3' splice junctions of at least one exon sequence of the dystrophin pre-mRNA, so that at least one exon sequence of the dystrophin pre-mRNA is skipped during the splicing process that converts the pre-mRNA into the mature mRNA.

2. The gene according to claim 1 in which the modified human U1 snRNA is further modified such that the U1 gene fragment transcribing the RNA sequence interacting to the U1 associated 70k protein is deleted.

3. The gene according to claim 1 or 2, wherein at least exon 51 of the dystrophin pre-mRNA is skipped.

4. A modified human U1 snRNA transcribed by the gene according to any one of claims 1 to 3.

5. A vector for stable and efficient expression in target cells expressing dystrophin transcribing the modified human U1 snRNA according to claim 4.

6. A vector according to claim 5 which is a retroviral, adenoviral or adeno-associated viral vector.

7. The vector according to claim 5 or 6 suitable for transfer to stem cells or to muscle fiber cells.

8. A gene according to any one of claims 1 to 3, a modified human U1 snRNA according to claim 4 or a vector according to any one of claims 5 to 7 for use in a method of treatment of the human or animal body by therapy.

9. A gene according to any one of claims 1 to 3, a modified human U1 snRNA according to claim 4 or a vector according to any one of claims 5 to 7 for use in the treatment of Duchenne Muscular Dystrophy.

10. Use of a gene according to any one of claims 1 to 3, a modified human U1 snRNA according to claim 4 or a vector according to any one of claims 5 to 7 in the manufacture of a medicament for use in the treatment of Duchenne Muscular Dystrophy.

## Patentansprüche

1. Gen, das für eine modifizierte humane U1 snRNA kodiert, wobei das U1 5' Genfragment, das die Region von U1 transkribiert, die einzelsträngig ist, eine Antisense-Sequenz umfasst, die zu den 5'- and 3'-Spleißverbindungen von mindestens einer Exon-Sequenz der Dystrophin prä-mRNA komplementär ist, so dass mindestens eine Exon-Sequenz der Dystrophin prä-mRNA während des Spleißprozesses übersprungen wird, die die prä-mRNA in die reife mRNA umwandelt.

2. Gen nach Anspruch 1, wobei die modifizierte humane U1 snRNA derart weiter modifiziert ist, dass das U1 Genfragment, das die RNA-Sequence transkribiert, die mit dem U1 assoziiertem 70k Protein interagiert, deletiert ist.

3. Gen nach Anspruch 1 oder 2, wobei mindestens Exon 51 der Dystrophin prä-mRNA übersprungen wird.

4. Modifizierte humane U1 snRNA, die von dem Gen nach einem der Ansprüche 1 bis 3 transkribiert wird.

5. Vektor für eine stabile und effiziente Expression in Zielzellen, die Dystrophin exprimieren, der die modifizierte humane U1 snRNA nach Anspruch 4 transkribiert.

6. Vektor nach Anspruch 5, der ein retroviraler, adenoviraler oder adeno-assoziierter viraler Vektor ist.

7. Vektor nach Anspruch 5 oder 6, der für eine Übertragung zu Stammzellen oder zu Muskelfaserzellen geeignet ist.

8. Gen nach einem der Ansprüche 1 bis 3, eine modifizierte, humane U1 snRNA nach Anspruch 4 oder ein Vektor nach einem der Ansprüche 5 bis 7 zur Verwendung in einem Verfahren zur Behandlung des menschlichen oder tierischen Körpers durch Therapie.

9. Gen nach einem der Ansprüche 1 bis 3, eine modifizierte, humane U1 snRNA nach Anspruch 4 oder ein Vektor nach einem der Ansprüche 5 bis 7 zur Verwendung in der Behandlung der Duchenne Muskeldystrophie.

10. Verwendung eines Gens nach einem der Ansprüche 1 bis 3, einer modifizierten, humanen U1 snRNA nach Anspruch 4 oder eines Vektors nach einem der Ansprüche 5 bis 7 in der Herstellung eines Medikaments zur Verwendugn in der Behandlung der Duchenne Muskeldystrophie.

## Revendications

1. Gène codant pour un ARNsn U1 humain modifié dans lequel le fragment du gène U1 5' transcrivant la région de U1 qui est simple brin comprend une séquence antisens complémentaires des jonctions d'épissage 5' et 3' d'au moins une séquence de l'exon du pré-ARNm de la dystrophine, de sorte qu'au moins une séquence de l'exon du pré-ARNm de la dystrophine soit omise pendant le procédé d'épissage convertissant le pré-ARNm en ARNm mature.

2. Gène selon la revendication 1, dans lequel l'ARNsn U1 humain modifié est davantage modifié de sorte que le fragment du gène U1 transcrivant la séquence d'ARN interagissant avec la protéine 70 k associée à U1 soit éliminé.

3. Gène selon la revendication 1 ou 2, dans lequel au moins l'exon 51 du pré-ARNm de la dystrophine est omis.

4. ARNsn U1 humain modifié transcrit par le gène selon l'une quelconque des revendications 1 à 3.

5. Vecteur pour l'expression stable et efficace dans des cellules cibles exprimant la dystrophine transcrivant l'ARNsn U1 humain modifié selon la revendication 4.

6. Vecteur selon la revendication 5, qui est un vecteur rétroviral, adénoviral ou viral adéno-associé.

7. Vecteur selon la revendication 5 ou 6, approprié pour un transfert vers les cellules souches ou vers les cellules fibreuses musculaires.

8. Gène selon l'une quelconque des revendications 1 à 3, ARNsn U1 humain modifié selon la revendication 4 ou vecteur selon l'une quelconque des revendications 5 à 7, pour une utilisation dans un procédé de traitement du corps humain ou animal par thérapie.

9. Gène selon l'une quelconque des revendications 1 à 3, ARNsn U1 humain modifié selon la revendication 4 ou vecteur selon l'une quelconque des revendications 5 à 7, pour une utilisation dans le traitement de la dystrophie musculaire de Duchenne.

10. Utilisation d'un gène selon l'une quelconque des revendications 1 à 3, d'un ARNsn U1 humain modifié selon la revendication 4 ou d'un vecteur selon l'une quelconque des revendications 5 à 7, pour la fabrication d'un médicament destiné à une utilisation dans le traitement de la dystrophie musculaire de Duchenne.
